# EUROPEAN PATENT APPLICATION

(11) **EP 2 345 645 A1**
(43) Date of publication of application: **20.07.2011**
(21) Application number: 09180507.7
(22) Date of filing: 22.12.2009
(51) Int. Cl.: C07D 263/20, C07D 413/06

(54) **Method for manufacturing stereoselective preparation of 4-bma using a chiral auxiliary and chiral auxiliary**

(71) Applicant: Savior Lifetec Corporation, 350 MiaLi County (TW)
(72) Inventor: Tseng, Wei-Hong, 351, Toufen Township, Miaoli County (TW); Chuang, Shiuan-Ting, 406, Beitun District, Taichung City (TW); Hung, Zun-Yuan, 608, Shuishang Township, Chiayi County (TW); Wu, Ching-I, 302, Hsinchu County (TW)
(74) Representative: Lang, Christian

(57) **Abstract**

The present invention relates to a process for preparing *(3R,4S)-3-*[[[R]-1 ' -t-butyldimethylsilyloxy ]ethyl]-4-[(R)-1 "-carboxyethyl]-2-azetidinone (beta- methylazetidin-2-one; 4-BMA), a key intermediate for the synthesis of carbapenem and penem antibiotics. Specifically, the present invention relates to a process comprising first, the preparation of a chiral auxiliary from cheap L-Phenylalaninol, and then the preparation of 4-BMA in high yield and high selectivity, under industrially mild condition.

## Description

### BACKGROUND OF THE INVENTION

The present invention relates to a manufacturing method of an intermediate for the synthesis of penems or carbapenems ,particularly A method for manufacturing an intermediate (3R,4S)-3- [[[R]-1 ' -t-butyldimethylsilyloxy ]ethyl]-4-[(R)-l "-carboxyethyl]-2-azetidinone (beta-methylazetidin-2-one; 4-BMA) and a chiral auxiliary.

4-BMA has been known in the art as an intermediate for the synthesis of 1 β-methylcarbapenem which exhibits potent antibacterial activity. Many types of carbapenems can be prepared from the 4-BMA,typical examples of which is Meropenem, Ertapenem, and Doripenem : 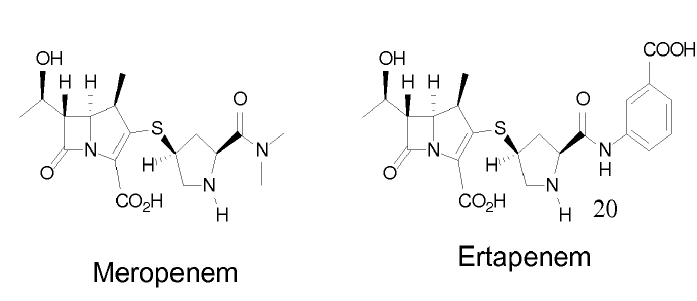 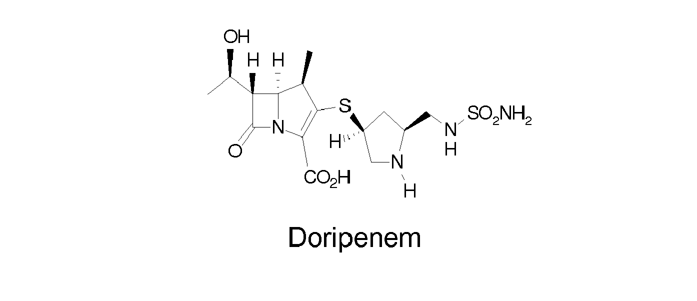 exhibits a broad spectrum of antibacterial activity against gram-positive and gram-negative strains. In particular, it has an excellent antimicrobial effect in controlling gram-negative strains and metalactamase- producing strains. Also, the presence of the beta-methyl group makes Meropenem, Ertapenem, and Doripenem to have better stability against dehydropeptidase-I (DRP-I) in the kidney compared to the existing carbapenem antibacterial agent of Imipenem (Antimicrobial Agents and Chemotheraphym 33, 215-222 (1984). Thus, in contrast to Imipenem, they does not have to be administered along with cilastatin to maintain stability in the body, and can be administered alone.

Various methods for preparing the 4-BMA, a key intermediate for manufacturing important medicines, such as carbapenem and penem antibiotics, have been developed. In earlier researches, 1 "-position hydrogen atom in the acetic acid residue at 4-position of the betamethyl compound was removed by a strong base, and methyl group was introduced thereto [Heterocycles, 21, 29(1984)]. However, this method posed problems of essentially using lithium diisopropylamide that is difficult to handle, and of having to be carried out under an extremely low temperature, such as -78°C. Thereis also the disadvantage that the compound having 1 a-methyl group of the following formula as followed 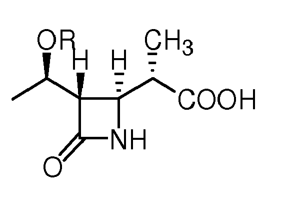 was produced in large amounts as a by-product (β/α=4/I). Several approaches have been tried to overcome such problems, and the most advantageous was to introduce p-methyl group using a chiral auxiliary. [Tetrahedron 52, 331-375, (1996)]

### <EARLIER METHODS FOR THE PREPARATION OF CHIRAL AUXILIARIES>

In most methods for preparing chiral auxiliaries for the synthesis of 4-BMA, propionyl group is introduced as an acyl group. A halide compound, which is not easy to handle, such as propionyl bromide, is used for introducing propionyl group, and a metal catalyst, such as n-butyllithimn, is used for the coupling reaction (JP2789190, DE3632916, US5104984, KR940008748, US5231179).

### <EARLIER METHODS FOR THE PREPARATION OF 4-BMA>

For the coupling reaction of (3R,4R)-4-acetoxy-3-[(R)-1 ' -(t-butyldimethylsilyl)oxy) ethyl]-2-azetidinone (4-AA) with the chiral auxiliary, trimethylchlorosilane (TMSCI) / lithium diisopropylamide (LDA), tintriflate [Sn(OTf)2],diethylborotriflate (Et2BOTf) / zinc bromide (ZnBr2), tert-butyldimethylsilyltriflate (TBDMSOTf) / zinc chloride (ZnCl2), LDA-Zr(Cp) 2Ch, etc. have been used (EP0974582, US5104984, J AM Chern. Sac, 1986, 108, 4675, etc.). However, these substances are explosive metal catalysts, or should be used in an extremely low temperature (-78 °C) reaction. Thus, it is difficult and uneconomical to use them industrially.

As summarized above, several methods for preparing 4-BMA have been reported, but a method suitable for preparing the desired compound in high yield and high selectivity using substances that are easy to handle in industrial production has not yet been developed.

### SUMMARY OF THE INVENTION

The primary object of the present invention is to provide a chiral auxiliary from cheap starting material in high yield under mild conditions, and in obtaining good quality of 4-BMA of β/α ratio.

Another object of the present invention is to provide a new process for preparing the 4-BMA that can be effectively used as an intermediate for preparing carbapenem or penem antibiotics.

Another object of the present invention is to provide a new process for preparing the chiral auxiliary effectively used for stereoselectively preparing the 4-BMA.

### BRIEF DESCRIPTION OF THE DRAWINGS

The structure and the technical means adopted by the present invention to achieve the above and other objects can be best understood by referring to the following detailed description of the preferred embodiments and the accompanying drawings, wherein
Fig. 1 is a schematic drawing showing chemical equation of Example 1
Fig. 2 is a schematic drawing showing chemical equation of Example 2a
Fig. 3 is a schematic drawing showing chemical equation of Example 2b
Fig. 4 is a schematic drawing showing chemical equation of Example 3a.3b
Fig. 5 is a schematic drawing showing chemical equation of Example 4a
Fig. 6 is a schematic drawing showing chemical equation of Example 4b

### DETAILED DESCRIPTION OF THE PREFERRED EMBODIMENT

A manufacturing method is described as following:

### Example 1: Preparation of (S)-4-benzyloxazoli dine-2-one-(2)

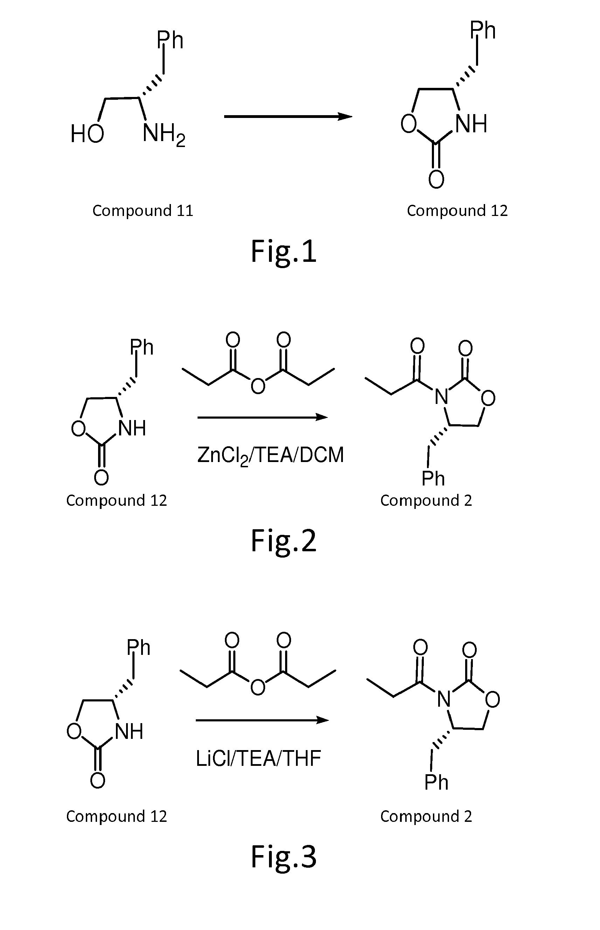

Refer to Fig 1, to a mixture of (2S)-2-amino-3-phenyl-1-propanol (649 g; 4.29 mol) and diethyl carbonate (1040 ml; 8.58 mol), anhydrous potassium carbonate (20 g; 0.14 mol) was added, and the mixture was stirred at 120 to 130°C for 3 hours. After cooling, to the resulting mixture 1 N hydrochloric acid (1.5 L) and ethyl acetate (about 20 L) were added and stirred. The organic layer was separated and washed with brine. Distilling off the solvent under reduced pressure gave (4S)-4-benzyloxazolidin-2-one (760 g, quantitative yield) as a colorless solid. The sample for analysis obtained by recrystallization from a mixed solvent of cyclohexane and toluene (1 : 1). Colorless crystals. M.P., 88 - 89°C. [α]²⁰ d -63° (c =1.0 in CHCl₃); IR (KBr): 1751, 1710, 1408, 1246, 1020, 944, 760, 710, 618, 532; ¹H-NMR (CDCl₃): δ7.33 (t, 3H), 7.26 (t,1H), 7.16 (d,2H), 5.39 (br, 1H), 4.44(t, 1H), 4.14 (dd, 1H), 4.07 (m, 1 H), 2.86 (d,2H); (IR, bs), 7.29 (SR, m); + Mass *m*/*e*: 177 (M); Calculated.: C, 67.78%; H, 6.26%; N, 7. 90 %. Found: C, 67.82%; H, 6.34%; N, 7.86 %

### Example 2a: Preparation of (S)-4-benzyl-3-propionyloxazoli dine-2-one-(2)

Refer to Fig 2, the compound (12) prepared in Example 1 (120g) was dissolved in methane dichloride (984ml), and cooled to 0°C. ZnC1₂ (52g) was added, triethylamine (101g) was then added, and the resulting mixture was stirred over a 30 min. Propionic acid anhydride (96.9g) was slowly added over a 30 min. time period. The reaction mixture was heated to reflux temperature and stirred for 1-1.5 hours. The reaction solution was cooled, water (300ml) was added, and the mixture was stirred for 30min. The methane dichloride phases were separated, and extracted once again with 1.5N hydrochloride solution (300ml). The organic solution was washed once again with aqueous 5% sodium bicarbonate solution (240ml). The organic solution was distilled by vacuum to remove methane dichloride until nothing come out. Heptanes was added to the resulting solution and stirred for 1 hours at - 5 to 0°C, which was then filtered and dried to produce the white solid compound (2) (150g, Yield 95%)
¹H -NMR 0 (CDC1₃): δ7.33 (dd, 2H) 7.30 (m, 1 H), 7.19 (d, 2H), 4.65 (m, 1H), 4.19-4.14 (m, 2H), 3.29 (dd, 1H), 3.00-2.89 (m, 2H), 2.75 (dd, 1H), 1 .19 (t, 3H)

### Example 2b: Preparation of (S)-4-benzyl-3-propionyloxazoli dine-2-one-(2)

Refer to Fig 3, the compound (12) prepared in Example 1 (120g) was dissolved in tetrahydrofuran (600ml), and cooled to 0°C. Lithium chloride (33.3g) was added, triethylamine (101g) was then slowly added, and the resulting mixture was stirred for 30 min. Propionic acid anhydride (96.9g) was slowly added over a 30 min. time period. The reaction mixture was slowly warmed to room temperature, and stirred for 1-1.5 hours. The reaction solution was cooled, 1N aqueous sodium chloride solution (300ml) was added, and the mixture was stirred for 30min. Ethyl acetate (300ml) was added, the phases were separated, and extracted once again by ethyl acetate (300ml). After washing with 1.5N hydrochloride solution (300ml), the organic solution was washed once again with aqueous sodium chloride solution (240ml). The ethyl acetate solution was distilled by vacuum to remove ethyl acetate until nothing come out. Heptanes was added to the resulting solution and stirred for 1 hours at 0-10°C, which was then filtered and dried to produce the white solid compound (2) (145.3g, Yield 92%)

### Example 3a: Preparation of (S)-3-((R)-2-(3-((R)-1-(t-butyldimethylsilyloxy)ethyl)-4-oxoazetidine-2-yl)propanoyl)-4-benzyloxazolidine-2-one-(10)

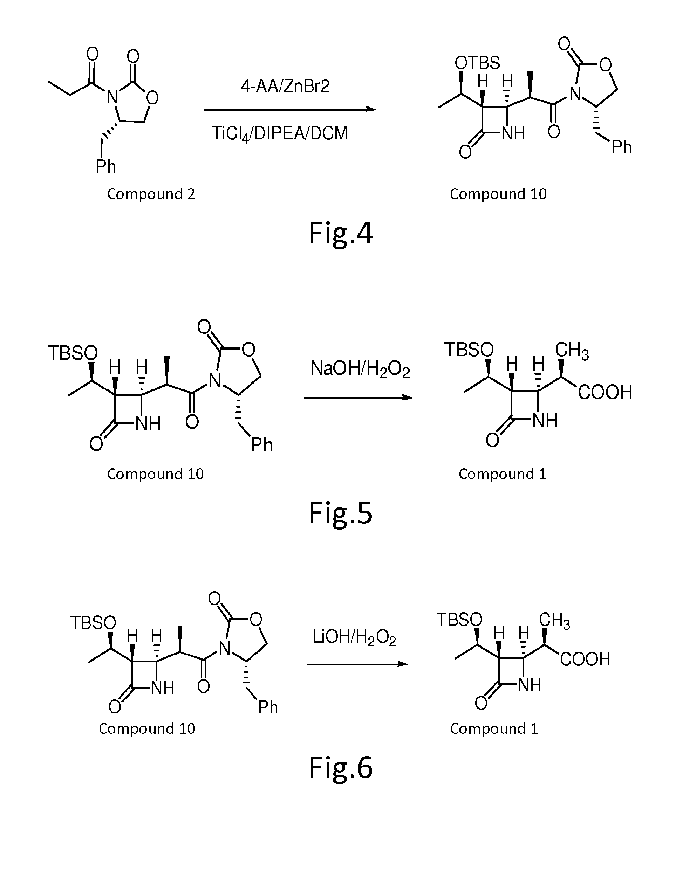

Refer to Fig 4,the compound (2) prepared in Example 2a or 2b (50.0g) was dissolved in methane dichloride (140ml), and cooled to 0°C. Titanium chloride (43.6g) was added. After 1 hour diisopropylethylamine (42.4g) and then Zinc bromide (24.1 g) in tetrahydrofuran (150ml) was added at 0 °C. Then 4-AA (50.0g) was added. The resulting mixture was reacted for 2 hours at 5-15°C. Water (300ml) was added to separate the phases. 1.5N hydrochloride acid (300g) was added thereto. The phases were separated and washed with aqueous sodium bicarbonate solution once again, distilled to produce the title compound contaminated with some impurities (98g).
^{l}H NMR (300MHz, CDCl₃) δ7.32 (m, 2H), 7.25 (m.1 H), 7.19 (d, 2H), 5.91 (s, 1 H), 4.66-4.63 (m, 1 H), 4.22-4.17 (m, 4H), 3.95 (m, 1 H), 3.30 (dd, 1 H), 3.06 (m, 1 H), 2.68 (dd, 1 H), 1.24-1.19 (m, 6H), 0.87 (s, 12H)

### Example 3b: Preparation of (S)-3-((R)-2-(3-((R)-1-(t-butyldimethylsilyloxy)ethyl)-4-oxoazetidine-2-yl)propanoyl)-4-benzyloxazolidine-2-one-(10)

Refer to Fig 4, the compound (2) prepared in Example 2a or 2b (50.0g) was dissolved in methane dichloride (140ml), and cooled to 0°C. Titanium chloride (43.6g) was added. After 30minute diisopropylethylamine (42.4g) was added at 0 °C. Then 4-AA (50.0g) was added. The resulting mixture was reacted for 4 hours at 15-20°C. Water (300ml) was added to separate the phases. 1.5N hydrochloride acid (300g) was added thereto. The phases were separated and washed with aqueous sodium bicarbonate solution once again, distilled to produce the title compound contaminated with some impurities (99g). ^{l}H NMR (300MHz, CDCl₃) δ7.32 (m, 2H), 7.25 (m.1 H), 7.19 (d, 2H), 5.91 (s, 1 H), 4.66-4.63 (m, 1 H), 4.22-4.17 (m, 4H), 3.95 (m, 1 H), 3.30 (dd, 1 H), 3.06 (m, 1 H), 2.68 (dd, 1 H), 1.24-1.19 (m, 6H), 0.87 (s, 12H) Example 4a: Preparation of (3R,4S)-3- [[ [R] -1' -t-butyldimethylsilyloxy] ethyl]-4- [(R)-1" -carboxyethyl] - 2-azetidinone (1)

Refer to Fig 5,the compound (10) prepared in Example 3a (98g) was dissolved in acetone (350ml) and water (200ml). Hydrogen peroxide (50ml) was added thereto, and the mixture was stirred at 0°C. Sodium hydroxide (19g) was dissolved in water (150ml), which was then added, over a 30 min. time period. The reaction solution was stirred for 1 hours at 15-25°C, white solid, the compound (12), was precipitated out. After filtering, washing by water (20ml) and drying the compound (12) was obtained that be reused to prepare compound (2) as example-2 (30.4g with 98.5% purity, 82% yield calculated from compound (2). Water (500ml) and methane dichloride (500ml) were added into resulting filtrate, and the phases were separated. The organic phase was distilled to produce the compound of formula (2) (20g). The aqueous phase was adjusted to pH 3.5 using 6N hydrochloric acid to produce a crystal. This crystal was filtered to produce the title compound. Then wet cake product was washed with the mixing solvent of methane dichloride and heptanes (50ml) at -5 to 5 °C. After drying at more than 40°C having ratio of >99.9/0.1 with 38.0g. (Yield 72% that was calculated from the 4- AA compound; purity 99.0% and assay 99.5% by HPLC). lH NMR (300MHz, CDC13)δ6.5 (br s, lH), 4.3 (m, lH), 3.97 (dd, lH), 3.05 (ddm,1H), 2.85 (m, lH), 1.29 (d , 3H), 1.22 (d , 3H), 0.89 (s, 9H), 0.08 (s,6H) [the corresponding □-isomer to 4-BMA] lH NMR (300MHz, CDC13)δ6.5 (br s, 1 H), 4.2 (m, lH), 3.72 (dd, lH), 2.85 (ddm,lH), 2.65 (m, lH), 1.3 (d , 3H), 1.23 (d , 3H), 0.90 (s, 9H), 0.08 (s ,6H)

### Example 4b: Preparation of (3R,4S)-3- [[ [R] -1' -t-butyldimethylsilyloxy] ethyl]-4- [(R)-I" -carboxyethyl] - 2-azetidinone (1)

Refer to Fig 6, the compound (10) prepared in Example 3a (98g) was dissolved in acetone (350ml) and water (200ml). Hydrogen peroxide (50ml) was added thereto, and the mixture was stirred at 0°C. Sodium hydroxide (19g) was dissolved in water (150ml), which was then added, over a 30 min. time period. The reaction solution was stirred for 1 hour at 15-25°C, white solid, the compound (12), was precipitated out. After filtering, washing by water (20ml) and drying the compound (12) was obtained that be reused to prepare compound (2) as example-2 (30.4g with 98.5% purity, 82% yield calculated from compound (2). Water (500ml) and methane dichloride (500ml) were added into resulting filtrate, and the phases were separated. The organic phase was distilled to produce the compound of formula (2) (20g). The aqueous phase was adjusted to pH 3.5 using 6N hydrochloric acid to produce a crystal. This crystal was filtered to produce the title compound. Then wet cake product was washed with the mixing solvent of methane dichloride and heptanes (50ml) at -5 to 5 °C. After drying at more than 40°C having ratio of >99.9/0.1 with 38.0g. (Yield 72% that was calculated from the 4- AA compound; purity 99.0% and assay 99.5% by HPLC).

The present invention has been explained by referring to the mode and the embodiments. However, the present invention is not limited to this mode and these embodiments, and can be modified or altered within the scope of the common knowledge of one having ordinary skill in the art.

## Claims

1. A method for manufacturing 4-BMA with a chiral auxiliary comprising the steps of: o o providing (s)-4-benzyl-3-propionylozazoli dine-2-one 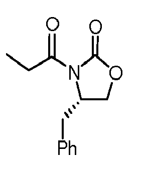 and an azetidinone compound 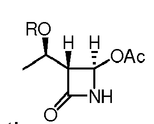 ;
adding titanium chloride in the presence of an organic base and a solvent of Lewis acid; and
hydrolyzing to form (3R, 4S)-3-[[[R]-1'-RO]ethyl]-4-[(R)-1"-carboxyethyl]-2-azetidinone 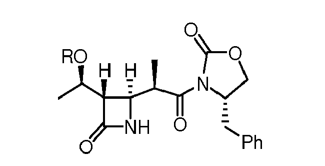 wherein R represents hydrogen or hydroxy-protecting group.

2. The method according to claim 1, wherein the organic base is selected from triethylamine (TEA), diisopropylethylamine (DIPEA), diethylamine (DEA) and butylamine

3. The method according to claim 1, wherein the Lewis acid is selected from lithium chloride (LiCI), aluminum chloride (AlCl₄), aluminum bromide (AlBr₄), iron tetrachloride (FeCl₄),zinc bromide (ZnBr₂), zinc chloride (ZnCl₂), trifluoroborane NiCl₂, BaCl₂, CoCl₂, MnCl₂. Ce(S04) ₂, Sml₂, NbCl₅, MoCl₅, B(OEt) ₃, ScCl₃, ReCl₅, YCl₃, VCl₃, TaCl₅, HfCl₄, ZrCl₄, AlCl₃ and SnCl₄.

4. The method according to claim 1, wherein the solvent is selected from dichloromethane, dichloroethane and chloroform.

5. The method according to claim 1, wherein the hydroxy-protecting group is an organic silyl group that is selected from the group consisting of t-butyldimethylsilyl, t-butyldiphenylsilyl, triethylsilyl. and trimethylsilyl.

6. The method according to claim 1, wherein the hydrolysis is carried out in the presence of hydrogen peroxide and lithium hydroxide or sodium hydroxid.

7. A method for manufacturing a chiral auxiliary for stereoselectively preparing 4-BMA comprising the steps of:
providing 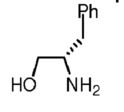 to mix with a base and diethyl carbonate;
and
forming 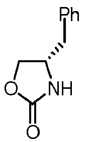 and reacting with propionic acid andydride in the
presence fo an organic base, a solvent and a Lewis acid.

8. The method according to claim 7, wherein the Lewis acid is selected from lithium chloride (LiCI), aluminum chloride (AlCl4), aluminum bromide (AlBr4), iron tetrachloride (FeC14),zinc bromide (ZnBr2), zinc chloride (ZnCl2), trifluoroborane.

9. The method according to claim 7, wherein the he organic base is selected from triethylamine (TEA), diisopropylethylamine (DIPEA), t-butylamine and diethylamine(DEA).

10. The method according to claim7, wherein the solvent is selected from tetrahydrofuran (THF), dimethylformamide (DMF), dimethylsulfoxide (DMSO), dimethylacetamide (DMAc) and acetonitrile (ACN).
